Europäisches Patentamt

European Patent Office

Office européen des brevets

(11) Publication number: **0 409 655 A2**

# EUROPEAN PATENT APPLICATION

(21) Application number: 90307983.8

(22) Date of filing: 20.07.90

(51) Int. Cl.5: **C12N 5/00**

(30) Priority: **21.07.89 US 383942**

(43) Date of publication of application:
**23.01.91 Bulletin 91/04**

(84) Designated Contracting States:
**AT BE CH DE DK ES FR GB IT LI LU NL SE**

(71) Applicant: **ORTHO PHARMACEUTICAL CORPORATION**
**U.S. Route 202 P.O. Box 300**
**Raritan New Jersey 08869-0602(US)**

(72) Inventor: **Sekine, Teruaki**
**1-1-13-420 Shiohama**
**Koto-Ku, Tokyo 135(JP)**

(74) Representative: **Mercer, Christopher Paul et al**
**Carpmaels & Ransford 43, Bloomsbury**
**Squareare**
**London WC1A 2RA(GB)**

(54) Method for stimulating proliferation of peripheral blood lymphocytes.

(57) This invention relates to a selective method of proliferation of at least one of certain lymphocytic cellular subpopulations, particularly the CD4[+], CD8[+], and Natural Killer (NK) subpopulations, within a starting population of peripheral blood lymphocytes (PBL). A relatively small amount of a starting peripheral blood lymphocytic cellular population is cultured in a multi-step process, beginning with an anti-CD3 antibody immobilized to a culturing substrate.

EP 0 409 655 A2

## METHOD FOR STIMULATING PROLIFERATION OF PERIPHERAL BLOOD LYMPHOCYTES

Field of the Invention

This invention relates to a selective method of proliferation of at least one of certain lymphocytic cellular subpopulations, particularly the $CD4^+$, $CD8^+$, and Natural Killer (NK) subpopulations, within a starting population of peripheral blood lymphocytes (PBL). A relatively small amount of a starting peripheral blood lymphocytic cellular population is cultured in a multi-step process, beginning with an anti-CD3 antibody immobilized to a culturing substrate.

Background of the Invention

Adoptive immunotherapy involves the administration of immunologically active cells to an individual. Such immunologically active cells are often referred to as natural killer cells (NK), lymphokine activated killer cells (LAK), and tumor infiltrating lymphocytic cells (TIL). A wide variety of human cancers currently have no effective treatment and are potential candidates for such cellular immunotherapy. However, one major drawback to such immunotherapy is the difficulty in obtaining the large numbers of cells that would be required for such treatment.

Various attempts have been made to augment the numbers of cells and the immunotherapeutic activity of the cells. The Immunobiology Research Center of the University of Minnesota in Minneapolis, Minnesota, reports the use of OKT3 plus IL2 to activate cultures of blood cells. They report an enhancement of the cell growth and LAK activity of peripheral blood monocytes when activated with this combination as opposed to activation of blood cultures with IL2 alone, i.e.: a 500-fold increase in cell number compared to a 7-fold increase for cultures activated with IL2 alone. "Augmentation of Cell Number and LAK in Peripheral Blood Mononuclear cells activated with Anti-CD3 and Interleukin-2" Cancer Immunology and Immunotherapy, Vol. 27:82-88 (1988).

International Application Publication No.: WO-88/00970 reports a method of culturing leukocytes to enhance overall LAK activity. A 30-100-fold increase in the number of cells was obtained. In a second aspect of the invention, leukocytes are cultured with a mitogen such as an anti-CD3 monoclonal antibody in the presence of interleukin-2. A 1000-fold expansion in cell number is obtained. In a further aspect, the leukocytes are further cultured in the presence of an additional lymphokine. The use of the anti-CD3 monoclonal antibody is by addition to a cell culture of the leukocytes, and the procedure seems to require multiple wash steps as the cells are cultured with the various mitogens and lymphokines. Tumor lytic activity, especially LAK activity, was apparently measured only in vitro .

Damle, N.K., et al., Journal of Immuno., Vol. 135, No. 3 (1985), pp. 1724-1730 report studies using a panel of monoclonal antibodies to examine the role of molecules on the surface of Leu-2$^+$ suppressor T cells. The Leu 2$^+$ subpopulation was first isolated, prior to treatment with Leu-4/T3 (CD-3) anti-CD3 monoclonal actibody. The authors postulate the involvement of such Leu-4/T3 (CD3) molecular complex, among others, found on the surface of the Leu-2$^+$ cells, in the activation and effector functions of suppressor T cells.

Geppert, Thomas and Peter Lipsky, Journal of Immuno., Vol. 138 (1987), pp. 1660-1666 report the use of immobilized anti-CD3 monoclonal antibodies to induce the proliferation of a purified population of T4 cells. The authors found that immobilized OKT3 was not very effective in stimulating T4 proliferation. They postulate that OKT3 only activates a subset of the total T4 cell population.

European Patent Application No. 0257962 reports the isolation of a natural killer population of cells and incubation of this subpopulation for activation.

Brief Description of the Figures

FIGURE 1 is a table depicting the major phenotypes of the peripheral blood lymphocytes proliferated according to the instant method, as compared to those of the control quantity.
FIGURE 2 is a graph depicting the expansion of the peripheral blood starting cell population as it is first cultured in vitro in the presence of anti-CD3 antibody immobilized onto a culture flask over a first period of time, then cultured in an uncoated culture flask over a second period of time, and then, finally cultured in a gas permeable bag over a third period of time.

Summary of the Invention

The present invention is directed to a multi-step method of stimulating the proliferation of peripheral blood lymphocytes and their activation and/or differentiation into at least one cellular subpopulation consisting of helper T cells, killer/suppressor T cells, or natural killer cells, by contact of a relatively small starting population of said peripheral blood lymphocyte population with an effective amount of an anti-CD3 antibody immobilized to a culture substrate for a first period of time, and secondly, contact of said same population with an uncoated culturing substrate for an additional period of time, said total culturing time period sufficient to induce the activation and/or differentiation and proliferation of such T cell subpopulations and/or natural killer cell populations in said sample of peripheral blood lymphocytes.

In more preferred embodiments, said same population, now greatly increased in cell number, is contacted in a third, expansion culturing step with a culturing substrate in the presence of a mitogen, prior to use in adoptive immunotherapy. In particularly preferred embodiments, said multiple contacting step with said separate culture substrates is carried out for a period of time sufficient to induce the proliferation of said starting cell population to greater than a 1000-fold increase in total cell number as compared to the starting cell population number.

The following definitions are taken from "Immunology" Roitt, I. et al., Gower Medical Publishing. London, New York (1985) and "Immunology: A Synthesis", E. Golub, Sinauer Ass., Inc. Massachusetts (1987).

As used herein, "helper T cells" are those cells having at least the T3 and T4 differentiation antigens, and are considered a functional subpopulation of lymphocytes.

As used herein, "killer/suppressor T cells" are those cells having at least the T3 and T8 differentiation antigens and are considered a functional subpopulation of lymphocytes.

As used herein, "natural killer cells" means leukocytic cells capable of recognizing cell surface changes on virally-infected cells. These cells may often be activated by interferons, perhaps produced by the virally-infected cells themselves. It is currently believed that this subpopulation of cells may be principally found in the null cell population of lymphoid cells, and may be of bone marrow origin.

As used herein, "anti-CD3 antibody" is an antibody, usually monoclonal in nature, that recognizes all or a portion of the CD3 complex, as that complex is described in the above references.

The advantages of the present method are many. For example, it is been found that the method is selective for proliferation of the $CD4^+$, $CD8^+$, and natural killer subpopulations from a starting peripheral blood lymphocyte cell population. Further, this proliferation is completely independent of monocytes. Increases in cell proliferation number obtained with the method of the invention as compared to the methods known to the art have demonstrated unexpectedly superior increases, in some cases as much as 30 times that obtained with the methods known to the art. Cytotoxicity of such activated cells for certain target tumor lines, such as K562, has also been increased. The need for mulitiple washing steps is also obviated through the use of the presently defined method.

Unexpectedly, with the multi-step in vitro cellular culturing according to the present method, the cell number is induced to increase many times the normal cellular proliferation number. Since this could be as much as four orders of magnitude or greater, a quantity of lymphocytes, immunologically activated, is provided that is suitable to render adoptive immunotherapy a viable technique.

Detailed Description of the Invention

Provided herein is a multi-step in vitro method to stimulate from a relatively small quantity of a starting population of peripherial blood lymphocytes, the proliferation of at least one distinct cellular subpopulation of "activated lymphocytes". As used herein, "activated lymphocytes" denote cells demonstrating natural killer activity (NK cells) capable of killing tumor cell lines; cells demonstrating tumor infiltration capabilities (TIL cells); and cells that demonstrate the ability to lyse fresh tumor and NK-resistant cell lines (LAK cells). "Activated lymphocytes" also denote lymphocytes with enhanced helper/inducer T cell and cytotoxic/suppressor T cell abilities, and cells displaying differentiation antigen characteristics of immunologically competent cells. The in vitro method is particularly selective for the proliferation of cells having the phenotype of helper/inducer T cells, cytotoxic/suppressor T cells, and natural killer cells.

A composition containing said enhanced subpopulation of cells, either alone, e.g.; helper T cells, or any combination of subpopulations, may be administered to patients (animal or human) in need of a large population of such cells for purposes of adoptive immunotherapy. An example of such patients are those with immunological disorders, in particular, cancer patients, patients demonstrating autoimmune disease, and the like.

In vitro stimulation of selective cellular proliferation according to the method of the invention is accomplished by contact of a relatively small amount of starting peripheral blood lymphocytes with a culturing substrate having immobilized thereto, an anti-CD3 antibody, and the subsequent contacting of said cell population with a second, uncoated, culturing substrate. A third contacting step is employed in the preferred embodiments. By such contact is generally meant a cellular culturing, as that term is accepted in the art.

To prepare a culture substrate for the first contacting step, an anti-CD3 antibody is immobilized to any conventional substrate suitable for such a purpose, including glass, nylon, polystyrene, polyurethane, polyolefin, and the like, as long as the antibodies are able to adhere in such a manner to the substrate to enable contact with surface molecules present on the peripheral blood lymphocytes. Preferred for use herein is an "uncoated " polystyrene culturing substrate, i.e.: one that is not treated for culturing. The substrate may vary widely in its overall configuration, but such configuration is preferred to be in a form that facilitates cell culturing techniques, for example, containers such as beakers, bottles, cell culture flasks, gas permeable cell culture bags, and the like. At the present time, untreated culture flasks, such as flasks conventionally used for suspension culture, are preferred.

Concentrations of the anti-CD3 antibody suitable for use herein vary from about 50 nanograms to about 5000 nanograms per $cm^2$ of immobilizing substrate. The antibody is first dispersed in any medium compatible with cell growth media. For example, the antibody may be dispersed in saline, phosphate buffered saline, methylcellulose, and the like. The pH of the resulting dispersion should also be physiologically compatible, and thus, preferably ranges from about pH 6.6 - pH 7.8, more preferably 6.7 to about 6.9. Culturing temperatures preferably range from about 35°C to about 38°C, more preferably 36.5°C to about 36.9°C.

The dispersion of anti-CD3 antibody is added to the immobilizing culture substrate (preferably a culture flask), and allowed to settle as homogeneously as possible onto the bottom, for immobilization thereto. One skilled in the art will understand when such immobilization has taken place when one notices that the surface of the culture substrate becomes smooth, most likely as a result of losing its hydrophobic nature. Usually a period of about 1-4 hours suffices for such immobilization. The antibody so immobilized may be stored at 4°C until use, and is stable for at least about 6 months at such storage conditions.

Prior to using the coated culturing substrate in the method of the invention, the anti-CD3 antibody-coated substrate may preferably be washed to remove debris or excess antibody. Any suitable washing media may be employed, as for example, phosphate buffered saline.

In preparation for the first step of the method of the invention, a relatively small quantity of blood (amounts as small as 10 ml) is treated to separate out a peripheral blood lymphocyte population. Treatments suitable for this purpose include separation techniques such as Ficoll-plaque density centrifugation and the like. Suitable amounts of a peripheral blood lymphocyte population are amounts as small as about $5 \times 10^5$ to about $3 \times 10^7$ PBLs.

The cell population so separated may be washed with any suitable media compatible with living cells. The seperated lymphocyte population is then suspended onto the culturing substrate (flask), previously coated with the anti-CD3 antibody, in any suitable tissue growth media, for example, minimum essential medium, RPMI 1640, and complete medium. The cell density of the cells so suspended should range from about $6 \times 10^3$ - $4 \times 10^5$ cells/$cm^2$ of anti-CD3 coated surface. From time to time, fresh media may be added to the cultured cells. This first period of culture with the immobilized anti-CD3 antibody generally ranges from about 2 to about 20 days.

In the second step of the method of the invention, and preferably after about 5-8 days, the cells from Step 1 may be transferred to a plain (uncoated) culturing substrate, for example, an uncoated culture flask, and allowed to continue culturing at approximately the same cell density in a culture medium such as the complete medium or any conventional equivalent. Said second culturing step is continued for a second 2 to about 30 day period, and preferably about 1 to about 10 days. While the present inventor does not wish to be bound theory, it is postulated at this time that this multi-step culturing provides an unexpectedly superior amount of differentiation and proliferation of certain cellular subpopulations. It can be ascertained from the art that high doses of immobilized anti-CD3 antibody could actually "inhibit" the growth of T cells, even in the presence of IL-2. Thus, it may be that in the past, culture of the cells in the same flask containing the immobilized antibody for an extended period of time, may actually serve to suppress the expansion of the PBL subpopulations, rather than enhance it.

Prior to the use of the proliferated cellular population for adoptive immunity and patient infusion, the cell culture containing the proliferated cell population obtained from the sequential culturing may be contacted in yet a further step with a lymphokine such as TNF, interferons ($\alpha$, $\beta$, or $\gamma$), and IL-2. The lymphokines may be added directly, or secretion of same by the cells themselves may be stimulated by the addition of a suitable amount of a conventional mitogen such as phytohemagglutinin, concanavalin A, and the like.

This third step may be referred to as the expansion culture step, which greatly enhances the activated cellular population to provide a sufficient quantity of cells for a therapeutic innoculum. The preferable lymphokine is IL-2 in concentrations ranging from about 200 U/ml to about 2000 U/ml. Suitable cell densities are relatively higher during this expansion culture, and may be in the order of $1.4 \times 10^6$ cells/ml. The cells are allowed to contact this lymphokine for a period of about 2-6 days, prior to harvest for patient infusion. A preferred cell culturing substrate for this third contact step is a conventional gas permeable cell culture bag, due to the need to maintain relatively high cell densities during this third culturing step. Cell culture bags are convenient and suitable for use in this regard, as oxygen may be supplied through the bottom. They are also economical, easy to keep sterile, disposable, and easy to work with in tight spaces, such as incubators. However, it should be appreciated that any culturing container capable of supporting the indicated cell densities, and capable of receiving various gas treatments ($O_2$, $CO_2$, etc.) could be substituted.

Here again, while the present inventor does not wish to be bound by theory, it is postulated that this third step encourages the unexpectedly superior yield of activated cellular subpopulations. Although various kinds of lymphokines are secreted from T cells through the stimulation by an anti-CD3 antibody, they are most likely actually washed away during the requisite washing steps in the prior art methods. The present multi-step method may offer an advantage by not requiring that the medium be changed during culture, which would tend to wash away secreted lymphokines. In the present method, one merely adds to the medium, and does not disturb the level of lymphokines. This maintenance of secreted lymphokines in the cell culture appears to be an added measure that enhances population growth.

The proliferated cells may be used for far-reaching therapeutic treatment of immunologically-based disorders. Whenever it would be suitable to employ an enhanced population of T4 cells, T8 cells, or the NK cells, whether alone or in combination, the method described herein provides a selective enhanced population of such cell types from a small amount of starting material, realistic for later therapeutic use. These cellular subpopulations are known for their cytotoxic properties, and in some cases, immunosuppressive properties. Natural killer cells are noted for their ability to non-specifically kill tumor cells and virally-infected cells, and also to play a role in regulating the immune response. This subpopulation of lymphocytes has been known to be suitable for use in various cancer treatments. Although it is not required, it is preferable that such an enhanced population be autologous to the patient undergoing treatment.

One skilled in the art will understand that conventional separation techniques may be employed to separate out a particular cellular subpopulation, e.g.: T8 cells, from the PBL population that has been greatly expanded through the method of the invention (if such a pure subpopulation of such cells is desired). Patients requiring treatment may be dosed with autologous or otherwise donor compatible cells vis a vis an intraveneous infusion regimen, bolus dosing, or by any other dosing method deemed appropriate. Cells treated by the method of the invention may be suspended in any compatible carrier such as phosphate buffered saline, methylcellulose, human albumin containing saline, and the like.

One salient example of the use of this enhanced population, is the ability to treat advanced metastic carcinoma patients (detailed in the Example section below). Tumor sites may vary widely, for example, skin, lymph node, abdomine, liver, lung, and the like. In these types of disease, activated lymphocytes in upwards of $10^8$ cells/ml are required for infusion, several times a week (see, for example, Rosenberg, et al., New England Journal of Medicine, Vol. 319, No. 25, pp. 1676-1680 (1988) for typical dosing regimes).

Such adoptive transfer treatment regimens are generally carried out in conjunction with a concomitant immunosuppression treatment of the tumor-bearing host. Such immunosuppression treatments may consist of total body irradiation, or concomitant dosing with drugs such as cyclophosphamide, peplomycin, cisplatin (CDDP), Fluorouracil (5-FU), Vindesine Sulfate (VDS), Decarbazine (DTIC). Patients may also be concomitantly dosed with a lymphokine such as IL-2, INFs, and TNFs.

While therapeutic uses have been described in some detail above, it should also be readily apparent that the proliferation method of the invention may be employed for uses other than therapeutic administration of the cell population so proliferated. For example, the large amount of normal human lymphocytes expanded by this method could be highly useful and efficient as a source of lymphokine production as well as a means of proliferation and/or purification of substances contained in the lymphocytes.

The following sets forth more specific embodiments of the present invention, intended as exemplary only, and not to be considered limitative thereof.

## EXAMPLES

In vitro stimulation of peripheral blood lymphocytes (PBLs) was carried out through the use of exposure to an immobilized monoclonal CD3 recognizing antibody, OKT3.

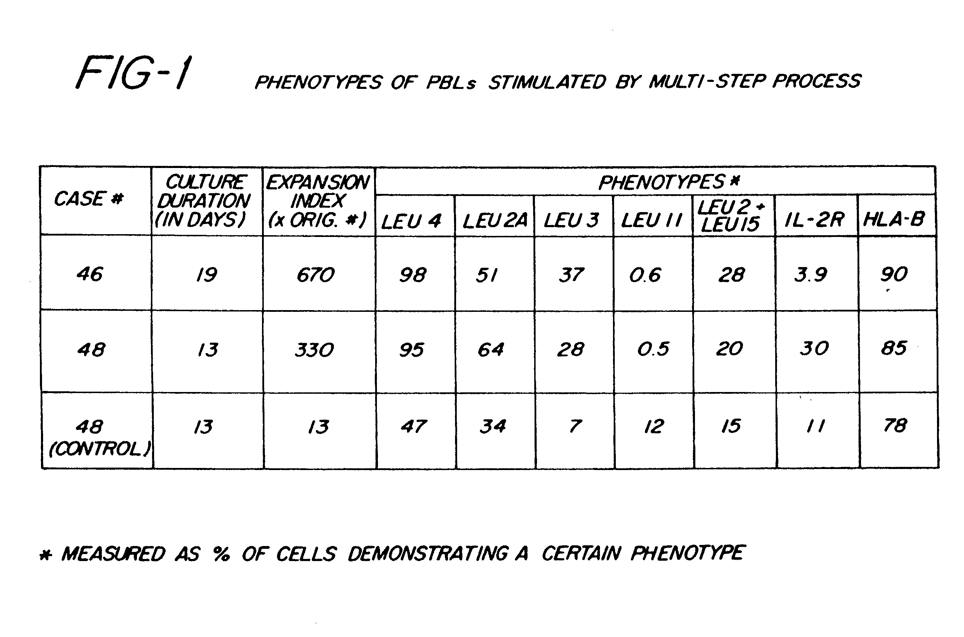

Peripheral blood lymphocytes (PBLs), obtained from 10ml whole blood, (approximately $1.4 \times 10^7$ PBL), were cultured in an OKT3-coated flask for 8 days. The cell number increased to 330 times the original starting cell number (See Figure 1). By contrast, the same number of PBLs cultured in a plain, uncoated flask for 8 days increased only 13 times the original cell number.

Phenotypes of the cultured PBLs in the OKT3-coated flask were different from the PBLs cultured in the plain, uncoated control flask. The major phenotypes of PBLs cultured in the OKT3-coated flask were $CD8^+$ and $CD4^+$ T cells, as depicted in the table shown in Figure 1. These phenotypes are indicative of cellular subpopulations effective in adoptive immunotherapy.

In a second experiment, PBLs were cultured in an OKT3-coated flask for 8 days, and then allowed to continue to culture in a plain, uncoated flask for an additional 21 days. The cell number increased to $1.7 \times 10^{11}$ (30,000 x that of the starting PBL cellular population). This greatly expanded amount of T cells was determined to be an amount sufficient for use in adoptive immunotherapy, especially since these cells were almost all T cells. The activated cells may be used in adoptive immunotherapy for advanced cancer patients.

In conclusion, a sufficient number of activated T cells for use in adoptive immunotherapy was obtained from a quantity as small as 10ml of starting whole blood, through the use of this multi-step culturing.

## Materials and Methods

The following is a more detailed description of the method used in these specific examples.

## Preparation of OKT3 coated culture flasks:

OKT3, 1-30 micrograms/ml, diluted with phosphatebuffered saline, pH7.4 (PBS) was poured into culture flasks, about 3ml for a $25cm^2$ flask, about 5ml for a $75cm^2$ flask, and 10ml for a $225cm^2$ flask. This solution was spread onto the bottom and allowed to settle at room temperature for a couple of hours. The coated flasks were stored in the refrigerator until use. Before use, coated flasks were washed with PBS three times.

## Culture:

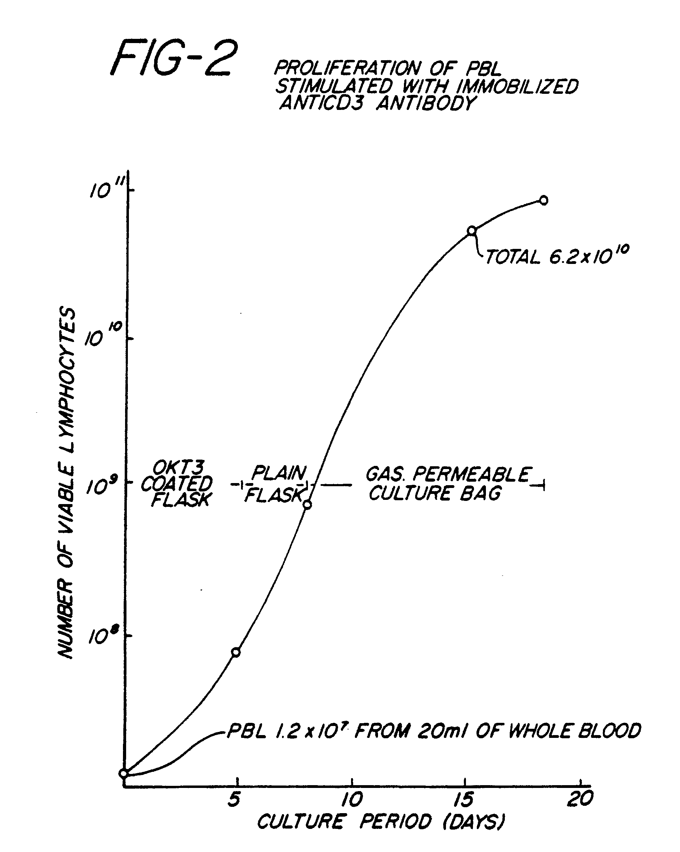

PBLs were separated from blood by Ficoll-paque density centrifuge and washed with RPMI-1640 medium two times, and suspended in complete medium at a cell density of about $1 \times 10^6$ cells/ml. Approximately 10ml to 20ml of the cell suspension was cultured in an OKT3-coated culture flask ($75cm^2$). During culture in the OKT3-coated flasks, the culture media turned to yellow. At that time, about 10ml of new complete medium was added. After about 3 to 8 days, cells were transfered to plain culture flasks and the culturing continued at a cell density of $1-2 \times 10^6$/ml in complete medium. In some cases. 3 days before adoptive immunotherapy infusion, the cultures were diluted with AIM-V medium containing 250 U/ml of IL-2 at a cell density of about $3-5 \times 10^5$ cells/ml,and separated to 1000ml per culture bag (Dupont, Stericell®). After 3 days of further culturing, cells were harvested. See Figure 2 for results.

Complete medium:
10% human serum (56° C 30 min. treated)
1mM sodium pyruvate
1mM oxalacetic acid
0.2 U/ml Insulin
60 microgram/ml Kanamycin
(50-100mM 2.mercaptoethanol, optionally added)
2mM L-glutamine
IL-2 1000 U/ml in RPM1-1640
Medium for Expansion Culture:
AIM-V (GIBCO)

1mM oxalacetic acid
1% serum
250-U/ml IL-2
(50-100mM 2-mercapto-ethanol, optionally added)


Culture flasks used for OKT3 coating:

Untreated culture flasks, such as flasks for suspension culture, were deemed to be better than the usual "adhesive" cell culture flasks, which are conventionally "treated" for cell culture.


Adoptive Immunotherapy Utilizing Enhanced PBL Population

Terminally ill cancer patients were treated with quantities of autologous PBLs, obtained from their whole blood samples subjected to the multi-step method of the invention for activation and expansion of cell number. Cell quantities used per infusion ranged from about $4 \times 10^{10}$ to about $16 \times 10^{10}$, such proliferated quantities obtainable in about 20 days of culturing with the method of the invention. The activated PBLs consisted principally of $T4^+$ and $T8^+$ cellular subpopulations. Greater than a 50% reduction in tumor size was evident in all patients treated with these activated cells, irrespective of the tumor site. The following table more specifically sets forth the data and results of this study:

| | Age | Diagnosis | Tumor | | Total Infusion Time |
|---|---|---|---|---|---|
| Case # | Sex | State | Site | P.S.* | Cell Number |
| 1 | 44 M | malignant melanoma IV | skin liver | 1 | 9 times 7.1 X $10^{10}$ |
| 2 | 57 M | malignant melanoma IV | lymphnode abdominal | 0 | 9 times 16.3 X $10^{10}$ |
| 3 | 46 F | malignant melanoma IV | skin | 0 | 3 times 4.2 X $10^{10}$ |

* P.S.: performance status

| | | After Infusion | | | |
|---|---|---|---|---|---|
| Case # | Concomitant Drugs | Response | | Present P.S. | Duration |
| | | Type | Site | | (month) |
| 1 | peplomycin IL-2 | PR NC | skin liver | 3 | Patient alive 5 months post treatment |
| 2 | CDDP VDS DTIC IL-2 | PR | lymphnode | 0 | Patient alive 5 months post treatment |
| 3 | ---- | PR** | skin | 0 | Patient alive 1 month post treatment |

** more than 50% reduced tumor size will be PR

## Claims

1. A sequential culturing method for selectively stimulating the proliferation of at least one individual subpopulation of T4 cells, T8 cells, and NK cells, from a relatively small quantity of a staring peripheral blood lymphocyte cell population, said method comprising the steps of:

a. culturing said relatively small quantity of peripheral blood lymphocytes with an effective amount of an anti-CD3 antibody immobilized onto a culturing substrate for a first period of time;

b. transferring said cell population so-cultured to an uncoated culturing substrate to continue culturing for a second period of time;

said cell population being allowed to culture for a total period of time sufficient to achieve proliferation of at least one of said individual subpopulations of T4 cells, T8 cells, and NK cells.

2. The method of claim 1 wherein said sequential culturing is for a total period of time sufficient to achieve at least about a 1,000-fold, preferably at least about 10,000-30,000 fold, combined increase in said cellular subpopulations as compared with said starting peripheral blood lymphocyte cell population.

3. The method of claim 1 or claim 2, wherein said starting peripheral blood lymphocyte cell population is about $10^6$ to about $10^8$ cells.

4. The method of any one of claims 1 to 3, wherein said anti-CD3 antibody is immobilized onto the surface of a culture flask.

5. The method of any one of claims 1 to 4, wherein the concentration of said anti-CD3 antibody immobilized onto said surface is about 50 nanograms/cm² of the surface to about 5,000 nanograms/cm² of the surface.

6. The method of any one of claims 1 to 5, wherein said anti-CD3 antibody is OKT3.

7. The method of any one of claims 1 to 6, wherein said first culturing step is for a period of about 3 to about 8 days, and said second culturing step is for a period of about 3 to about 21 days, preferably about 3 to about 5 days.

8. The method of any one of claims 1 to 7, further comprising, after the step b., the step of: c. transferring said cell population to a third culturing substrate in the presence of a lymphokine, for a period of time ranging from about 2 to about 5 additional days, preferably about 2 to about 4 days, preferably in a gas permeable culture bag.

9. The method of claim 8, wherein said lymphokine is IL-2, TNF, $\alpha$-interferon, $\beta$-interferon, or $\gamma$-interferon, preferably Il-2.

10. The method of any one of claims 1 to 9, wherein said culturing temperature is maintained between about 36.5° C and 36.9° C and said pH is maintained between about pH 6.7 and pH 6.9.

## FIG-1  PHENOTYPES OF PBLs STIMULATED BY MULTI-STEP PROCESS

| CASE # | CULTURE DURATION (IN DAYS) | EXPANSION INDEX (x ORIG. *) | PHENOTYPES * | | | | | | |
| --- | --- | --- | --- | --- | --- | --- | --- | --- | --- |
| | | | LEU 4 | LEU 2A | LEU 3 | LEU 11 | LEU 2 + LEU 15 | IL-2R | HLA-B |
| 46 | 19 | 670 | 98 | 51 | 37 | 0.6 | 28 | 3.9 | 90 |
| 48 | 13 | 330 | 95 | 64 | 28 | 0.5 | 20 | 30 | 85 |
| 48 (CONTROL) | 13 | 13 | 47 | 34 | 7 | 12 | 15 | 11 | 78 |

\* MEASURED AS % OF CELLS DEMONSTRATING A CERTAIN PHENOTYPE

FIG-2  PROLIFERATION OF PBL STIMULATED WITH IMMOBILIZED ANTICD3 ANTIBODY